# EUROPEAN PATENT APPLICATION

(11) **EP 4 427 710 A1**
(43) Date of publication of application: **11.09.2024**
(21) Application number: 24162105.1
(22) Date of filing: 07.03.2024
(51) Int. Cl.: A61F 2/58, A61F 2/76, A61F 2/78, A61F 5/01, A61F 2/50, A61F 2/54, A61F 2/72

(54) **RATCHET SYSTEM AND ORTHOTIC SYSTEM**

(30) Priority: 07.03.2023 EP 23160455
(71) Applicant: macu4 AG, 8001 Zürich (CH)
(72) Inventor: SCHILLER, Lukas, 8006 Zürich (CH); CHEVROT, Alec, 1002 Chavannes-près-Renens (CH)
(74) Representative: Schulz Junghans Patentanwälte PartGmbB

(57) **Abstract**

The present invention relates to a ratchet system (90), comprising a first part (300) of the ratchet system (90) equipped with a first serration (91) and a second part (301) of the ratchet system (90) equipped with a second serration (92), wherein the two serrations (91,92) comprise serration elements (93) that are at least partially complementary in terms of shape, number, and/or size, and wherein the first part (300) of the ratchet system (90) has a retainer (82) with an insertion opening (83) and is equipped with the first serration (91), and the second part (301) of the ratchet system (90) has an insertion element (110) equipped with the second serration (92), such that after insertion of the insertion element (110) through the insertion opening (83) along a slide-in direction (111) into the retainer (82), the two serrations (91,92) can interlock and can block at least one translational degree of freedom of the insertion element (110) along the slide-in direction (110) with respect to the retainer (82).

Further, the present invention relates to an orthotic system, comprising the ratchet system and an orthotic accessory.

## Description

The present invention relates to ratchet system and to an orthotic system, comprising the ratchet system.

In the case of a congenital deformity or amputation, it may be difficult to perform bimanual activities such as fitness training or kayaking. Similarly, holding a shopping bag or walking on crutches can become a challenge. Daily life for these people is full of little challenges that an ordinary person would never suspect, let alone imagine. People with a birth defect learn to live with it, but outside help is welcome to ease the effort and mental burden. For people who have suffered an amputation as a result of an accident, for example, it is necessary to put a relearning of skills in place. Tools can facilitate not only their daily life but also their rehabilitation. Similarly, in the event of an accident, surgery or injury, hands may become weakened, sensitive, immobilized for a limited period. Therefore, a rapid provision of support is essential to enable affected individuals to continue their activities and daily routines.

Especially if a person has a double disability and is dependent on crutches, any hand restriction can lead to a complete loss of mobility, as the person cannot use conventional crutches without their hands.

An orthotic system is designed for individuals with partial or complete loss of hand function. This loss may be due to temporary or permanent weakening, hand injury, paralysis, neuromuscular disease, partial absence of the hand or absence due to wrist disarticulation.

An orthotic system designed for forearms is intended to assist individuals with a disability below the wrist. This encompasses various scenarios, including those with only a portion of the palm remaining, small residual fingers, a limited number of fingers, or limited and painful movement capacity. In consequence, the system should be engineered to transmit a force generated by a module directly to the forearm, wherein the connection system allows to connect different kind of modules.

There is a need for an orthotic system allowing to hold handles, bars, rods or similar structures and to transmit a tensile or compressive load from the object to the forearm. Such orthotic system should enable the use of different types of crutches.

Existing solutions are often poorly adapted, expensive and restricting in terms of the activities they support.

The problem to be solved by the present invention is to provide a ratchet system and an orthotic system that ensure comfort through a lightweight, intuitive design while effectively transferring load forces from and/ or to a body of a user, wherein different kind of modules for different activities can be connected.

This problem is solved by the subject-matter of the independent claims 1 and 12, wherein claim 1 deals with a ratchet system and claim 12 deals with an orthotic system.

Embodiments of the ratchet system are claimed in dependent claims 2 to 11.

Embodiments of the orthotic system are claimed in dependent claims 13 to 15.

A first aspect of the invention is a ratchet system, comprising a first part of the ratchet system equipped with a first serration and a second part of the ratchet system equipped with a second serration, wherein the two serrations comprise serration elements that are at least partially complementary in terms of shape, number, and/or size. The first part of the ratchet system has a retainer with an insertion opening. The retainer is equipped with the first serration, and the second part of the ratchet system has an insertion element equipped with the second serration, such that after insertion of the insertion element through the insertion opening along a slide-in direction into the retainer, the two serrations can interlock and can block at least one translational degree of freedom of the insertion element along the slide-in direction with respect to the retainer.

When the serrations of the first and second part of the ratchet system are brought in a state of interlocking, a positive locking is established, enabling a load transfer along the slide-in direction between the first part of the ratchet system and the second part of the ratchet system.

The first part of the ratchet system and the second part of the ratchet system can repeatably be connected and disconnected.

Thereby, the insertion element can repeatedly be inserted into and removed from the retainer. The insertion element is inserted along the slide-in direction and pulled out in the opposite direction of the slide-in direction. The insertion opening may be an insertion slot.

When the insertion element is inserted into the retainer, the retainer can block at least one translational degree of freedom of the insertion element perpendicular to the slide-in direction A serration can be an arrangement of serration elements, each having a form that comprises an undercut. These serration elements, along with their respective undercuts, can be arranged next to each other to form recesses between the serration elements. In one embodiment the serrations may be seen as an arrangement of notches.

The complementary shapes, numbers, and/or sizes of two serrations allow the individual serration elements to interlock wherein a serration element is moved in a respective recess, enabling the engagement of the two serrations. Serrations elements can taper to a point or can have a rounded shape.

The serration of the first part and the second part of the ratchet system may have distinct designs, yet comprising serration elements that are at least partially complementary in terms of shape, number, and/or size. This means even though the designs may be different, the serration elements still are able to engage with each other due to their complementary features.

In one alternative embodiment, the serration is realized with a pin and hole system, wherein the first part of the ratchet system comprises at least one hole and the second part of the ratchet system comprises at least one pin, designed to be inserted in the hole and thereby establishing a positive locking.

The first part of the ratchet system comprising the retainer can be a part of an orthotic accessory which is or can be attached to a limb or can be connectable with such orthotic accessory. The second part of the ratchet system comprising the insertion element can be a part of a module or can be mechanically connected to a module, whereby the module is designed to be connected to the orthotic accessory by means of the ratchet system.

In one embodiment of the ratchet system, the first serration at the first part of the ratchet system and second serration at the second part of the ratchet system comprise a set of teeth, respectively.

The insertion element can be characterized in that it has a transversal extension and a longitudinal extension longer than the transversal extension and can be inserted along its longitudinal extension into the retainer, wherein the retainer comprises at least two opposing surfaces, which enclose or can enclose the insertion element, such that at least one translational degree of freedom of the insertion element perpendicular to the slide-in direction with respect to the retainer is at least partially blocked or can be at least partially blocked by the retainer.

Partially blocked can mean, that sections of the insertion element can still move within the respective translational degree of freedom, like for example the section, that is equipped with the second serration.

The retainer can also block one translational degree of freedom of the insertion element perpendicular to the slide-in direction, while a second translational degree of freedom perpendicular to the slide-in direction is only partially blocked. Said second translational degree of freedom can be perpendicular to both, the slide-in direction and the first translational degree of freedom.

A plurality of serration elements of the first and second serration may be arranged in rows, wherein the first and the second part of the ratchet system comprise at least one row, respectively.

The serration elements of a row essentially are aligned in a manner, that the orientation of the undercut of at least some of the serration elements is perpendicular to the slide-in direction. This results in the engagement of the serration elements being able to effectively block the translational degree of freedom of the insertion element along the slide-in direction with respect to the retainer.

Such a row can be an arrangement in a straight line, parallel to the slide-in direction. The number of serration elements in the rows of the first serration and the second serration may differ.

Furthermore, the first serration and the second serration can have serration elements that are arranged along the slide-in direction in such a way that more than one locking position and/or insertion length of the insertion element in the retainer can be realized along the slide-in direction.

The adjustment can be executed by disengaging, repositioning and relocking the first and the second serration. The disengaging can also be referred to as releasing, unlocking or uncoupling the engagement. The repositioning can also be referred to as changing the locking position or sliding the insertion element.

The different locking positions can allow for different incremental positions of the insertion element along the slide-in direction.

To ensure different locking positions are enabled, the number of serration elements of the first serration and number of serration elements of the second serration can be different. For instance, a first row of serration elements provided by the first serration can be longer than a respective second row of serration elements provided by the second serration. As a result, the insertion element can be locked at different positions along the slide-in direction with respect to the retainer.

As a consequence of multiple locking positions, the longitudinal extension of the ratchet system can be adapted through adjusting the locking position between the first part of the ratchet system and the second part of the ratchet system.

Another embodiment is characterized in that the insertion element can at least partially be displaced and/or deformed due to the application of a pressing force induced manually to the insertion element, so that an engagement of the first serration and the second serration can be prevented or dissolved.

At least a component of the pressing force can be perpendicular to the slide-in direction.

Due to the disengagement of the first serration element and the second serration element, the insertion element can be moved within the retainer along the slide-in direction or in the opposite direction of the slide-in direction. The mechanism of adjusting the locking position can be referred to as press and slide, press and change, press and push or release and lock.

Moreover, the insertion element may be removed from the retainer by pulling it out in the opposite direction of the slide-in direction. As a result, a separation of the first part of the ratchet system and the second part of the ratchet system can be enabled.

Additionally, the insertion element can be adapted in such a way that a manually applied pressing force of at least 3 N elastically bends and thus displaces at least a section of the insertion element and thereby prevents or dissolves the engagement between the first serration and the second serration, wherein the Young's modulus of the insertion element is between 0.5 GPa and 7 GPa.

When the insertion element is bent by the pressing force, the retainer may be configured to absorb the induced bending moment.

In case the insertion element is made of a metal or a composite, the Young's modulus of the insertion element may be between 50 GPa and 500 GPa.

Furthermore, the insertion element can comprise an optically and/or haptically recognizable button, designated for applying the pressing force in the insertion element in order to displace and/or deform the insertion element at least partially, thereby preventing or dissolving the engagement between the first serration and the second serration.

In this embodiment, the button has at least two positions, an unlocked position where the insertion element can be moved within the retainer and a locked position where the serration elements of the first serration are engaged with the serration elements of the second serration. In the locked position, the movement of the insertion element along the slide-in direction can be locked.

Said button can be an integral part of the insertion element. The button can be provided in the end section of the insertion element which is adapted to be pushed into the retainer.

Moreover, the button may provide a button cavity for applying a force along the slide-in direction into the insertion element, enabling to move the insertion element along the slide-in direction after disengaging the first and the second serration.

The button can further have a structured surface to increase the friction between the button and a person's finger, pressing the button. An increased friction can be advantageous when applying a force along the slide-in direction into the insertion element.

In one embodiment, the ratchet system is characterized in that the second serration provided by the insertion element comprises two rows of serration elements, that are aligned parallel to one another and parallel to the slide-in direction. The button is located in between these two rows. Furthermore, the first serration provided by the retainer also comprises to rows of serration elements that are parallel to one another and parallel to the slide-in direction.

The insertion element can be provided at least partially by a material having a Young's modulus between 0.5 GPa and 7 GPa so that after elastic deformation of the insertion element due to elastic behaviour of the insertion element at least one section of the insertion element is movable such that the second serration of the insertion element is brought into positive locking with the first serration of the retainer.

This implies that the insertion element is designed, due to an application of force, to bring the first part and second part of the ratchet system in a positive locking. The application of force can be realized by the elastic restoring force of the deformed elastic material of the insertion element.

The insertion element may be partially bent while inserting the insertion element into the retainer, as the insertion elements adapts its geometry to fit into the retainer. Consequently, the insertion element is in a state of bending stress. Thereby the retainer is configured to absorb the induced bending moment. Once the first serration is aligned with the second serration, the bending stress can be partially unsupported and pushes the first serration into positive locking with the second serration, wherein the bending stress is reduced or eliminated at least minimized.

In case the insertion element is made of a metal or a composite, the Young's modulus of the insertion element may be between 50 GPa and 500 GPa.

An embodiment of the ratchet system is characterized in that the insertion element has a bridging section, comprising the second serration and a first and second support element located ahead and behind the bridging section in relation to the slide-in direction for supporting the insertion element on support parts provided by the retainer. The support elements can protrude at an angle from the bridging section with respect to the slide-in direction, respectively, wherein the first and second serrations are configured such that their serration elements are interlocked in an engaged state in a plane of engagement. The retainer forms an enclosure for the insertion element, blocking at least one rotational degree of freedom of the inserted insertion element around an axis perpendicular to the slide-in direction, wherein the bridging section and/or the support element located at the end of the bridging section are provided by a material having a Young's modulus between 0.5 GPa and 7 GPa, so that when a manual force is applied to the bridging section, the bridging section and/or said support element is able to elastically deform, resulting in the disengagement of the first and second serrations.

The protruding angle of at least one support element can be in a range of 20° to 160° to the plane of engagement. The axis of the restricted rotational degree of freedom of the insertion element is parallel to the plane of engagement of the first and second serration and perpendicular to the slide-in direction.

Furthermore, the support elements can be arranged in such a way, that the insertion element has a portal-shaped form.

In one embodiment, the support parts of the retainer may be integral parts of one surface of the retainer.

The support elements protruding from the bridging section do not necessarily need to be directly connected to the bridging section, but can also be connected to an element, that is mechanically connected to the bridging section or is an integral part of the bridging section.

The support element located at the end of the bridging section may also indicate that the support element is located at the end of the insertion element.

In case the insertion element is made of a metal or a composite, the Young's modulus of the insertion element may be between 50 GPa and 500 GPa.

Alternatively, the ratchet system can be an interface which comprises a first connection element, designed to mechanically connect the first part of the ratchet system to an orthotic accessory connectable to a person's limb or designed to mechanically connect the first part of the ratchet system to a module for establishing a connection to an object. Furthermore, the ratchet system can comprise a second connection element, designed to mechanically connect the second part of the ratchet system to the respective other part of orthotic accessory or module, in order to enable, that the orthotic accessory and the module can be repeatable connected and disconnected.

Such an interface can be used by professionals to be integrated into custom-made orthotic system. In one embodiment, the first connection element is used to be integrated into limb accessories, for example cuffs and the second connection element can be used to be integrated into a module which is an orthotic device.

The integration of both connection elements can be seamless or by means of any other mechanical connection.

The connection elements can be a protrusion, respectively, providing a surface that can be glued to or embedded into an orthotic accessory or orthotic device. The connection elements may also comprise bores, designed for a screwed connection of the ratchet system to the orthotic system or the orthotic device.

A further aspect of the present invention is an orthotic system, comprising a ratchet system and an orthotic accessory connectable to a limb.

The orthotic accessory can be part of a cuff, connectable to a limb.

An orthotic system, comprising the ratchet system and a cuff can be a convenient system for establishing a connection to a limb. For instance, the first part of the ratchet system can be a part of the cuff and the second part of the ratchet system can be a part of a module. This allows to connect the module to the cuff by means of the ratchet system and therefore to establish a connection between the module and the limb.

One embodiment of the cuff comprises a cuff base in form of a lower shell and a fixation element which can be combined or is combined with the lower shell into a sleeve attachable to a limb through direct or indirect interlocking with each other, wherein the lower shell and the fixation element provide a system that can be tightened around the limb by reducing a circumference formed by the lower shell and the fixation element, such that load transfer from an accessory to the limb is enabled.

The insertion of a limb, for instance a forearm, into the cuff can be difficult as, in most cases, there is an enlargement at the beginning of the palm. In order to facilitate the insertion of the cuff, in this embodiment the cuff design is divided into a cuff base in form of a lower shell and a fixation element, which can be a cuff top in form of an upper shell, or a strap system.

The possible reduction of the circumference formed by the lower shell and the fixation element, for example, with a strap system or a lacing system can provide a fast and comfortable way to interlock the cuff with the forearm.

Such a system can ensure that a load can be transferred from the cuff to the forearm or from the forearm to the cuff. When the cuff is further connected to a module, the load can be also transferred to or from the module.

In one embodiment of the orthotic system, the second part of the ratchet system is provided by a device for establishing a form-fitting and/or force-fitting connection to an object that comprises a mechanical holding mount, where the object can be positioned, such that load transfer from the object to the limb is enabled, and at least one belt mechanically connected to said holding mount. The belt is configured to be partially wrapped around the object positioned in the mechanical holding mount in order to fix the object in at least one translational degree of freedom.

Such an orthotic system can be used to perform tasks that typically require the use of hands. A person with hand impairment or without hands can benefit from the device in combination with the cuff. For instance, the person can use crutches by connecting the device to the handles of the crutch via the mechanical holding mount.

At least one of the elements of the orthotic system may be manufactured by additive manufacturing.

The invention is illustrated by the following examples and figures, from which further embodiments and advantages can be drawn. These examples are meant to illustrate the invention but not to limit its scope.

It is shown in
Fig. 1 : a first embodiment of the ratchet system in a perspective view,
Fig. 2: a top view of the ratchet system shown in fig. 1,
Fig. 3: a cross sectional view of the ratchet system shown in fig. 1,
Fig. 4: a part of the cuff as well as a part of the device in a first state,
Fig. 5: the part of the cuff as well as the part of the device in a second state,
Fig. 6: the part of the cuff as well as the part of the device in a third state,
Fig. 7: the part of the cuff as well as the part of the device in a fourth state,
Fig. 8: a first embodiment of the device in a perspective view,
Fig. 9: a second embodiment of the device in a perspective view in a first state,
Fig. 10: perspective view of the device shown in fig. 9 in a second state,
Fig. 11: a first embodiment of the cuff in a perspective view,
Fig. 12: a second embodiment of the cuff in a perspective view,
Fig. 13: the underside of the cuff shown in fig. 11, and
Fig. 14: an orthotic system with the cuff and module in a perspective view.

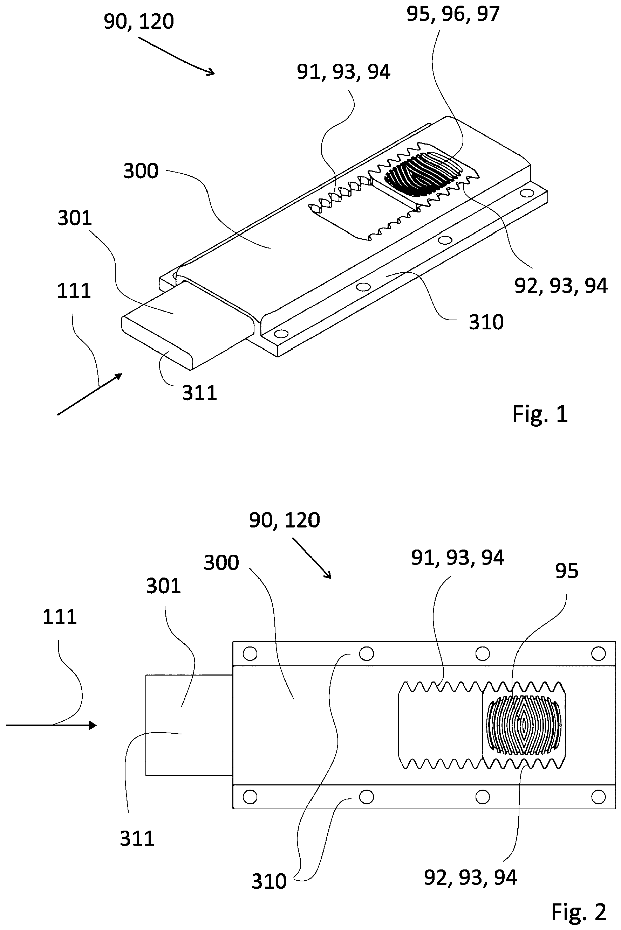

Fig. 1 shows a ratchet system 90 in a perspective view in accordance to the invention. The ratchet system 90 comprises a first part 300 of the ratchet system 90 and a second part 301 of the ratchet system 90.

The first part 300 of the ratchet system 90 is equipped with a first serration 91 with serration elements 93 in the shape of teeth 94. The second part 301 of the ratchet system 90 is equipped with a second serration 92 with serration elements 93 that are complementary in form and size to the teeth 94 of the first part 300 of the ratchet system 90.

The teeth 94 of both serrations 91,92 are arranged in two rows, respectively, which are in a state of positive locking 120 with each other. Thereby, the teeth 94 of both serrations 91,92 are positioned in respective recesses formed between the teeth 94 of the respective other serration 91,92, enabling the positive locking 120 and further enabling a transmission of load between the first and second part 300,301 of the ratchet system 90.

Moreover, the shown embodiment comprises a button 95 as part of the second part 301 of the ratchet system 90, wherein the button 95 is arranged between the two rows of teeth 94 of the second serration 92. The row of teeth 94 are facing into opposite directions, pointing sideways away from the button 95.

The button 95 is designed to be manually pushed against an elastic force of the second part 301 of the ratchet system 90. The second serration 92 can be disengaged from the positive locking 120 in a convenient way by pressing the button 95.

The button 95 comprises a button cavity 96 and a structured surface 97 in order to facilitate the pressing and moving of the button 95 with a person's finger along a slide-in direction 111.

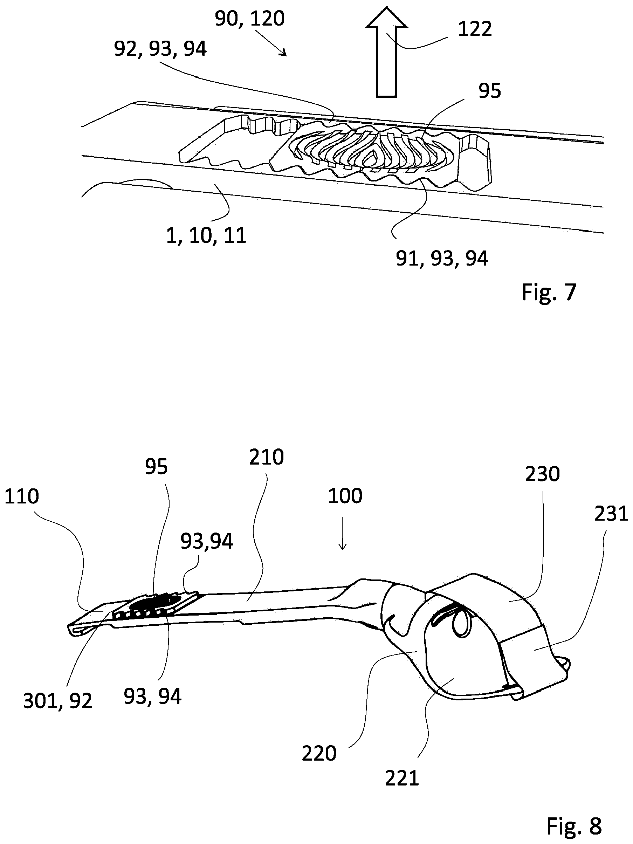

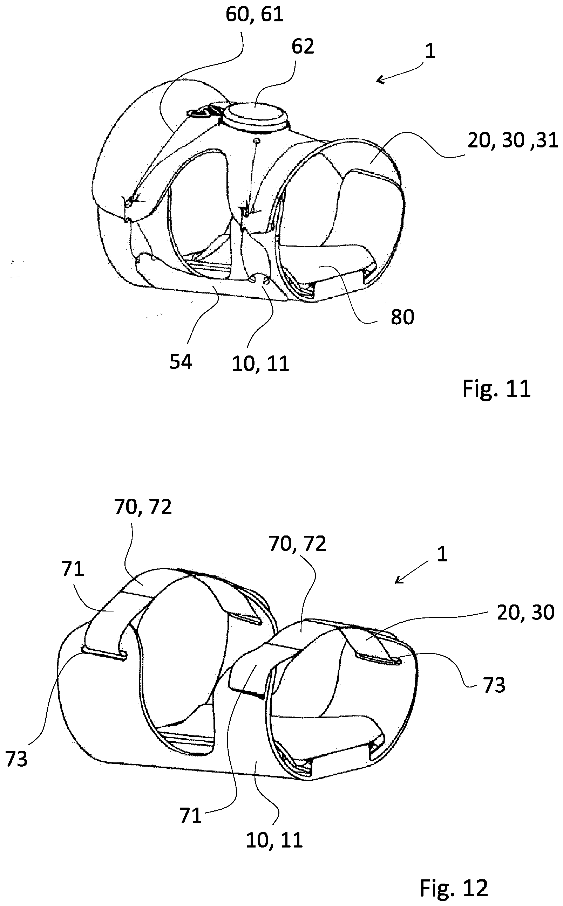

The ratchet system 90 in fig. 1 further shows a first connection element 310 and a second connection element 311. The first connection element 310 comprises several bores, that can be used to connect the first part 300 of the ratchet system 90 to an orthotic accessory, designed to establish a connection to a person's limb, like for instance a cuff. The second connection element 311 can be used to connect the second part 301 of the ratchet system 90 to a module, like for example a device for establishing a form-fitting or force-fitting connection to an object. This enables a connection that can be repeatably dissolved between the orthotic accessory and the module, utilizing the ratchet system 90 as an interface. A module can be a device 100 as shown in fig. 8 to 10. An orthotic accessory can be cuff 1 as shown in fig. 11 to 14.

Fig. 2 shows a top view of the ratchet system 90 in the same embodiment as in fig. 1. As can be seen here, the first connection element 310 of the interface comprises two lateral protrusions with four bores, respectively.

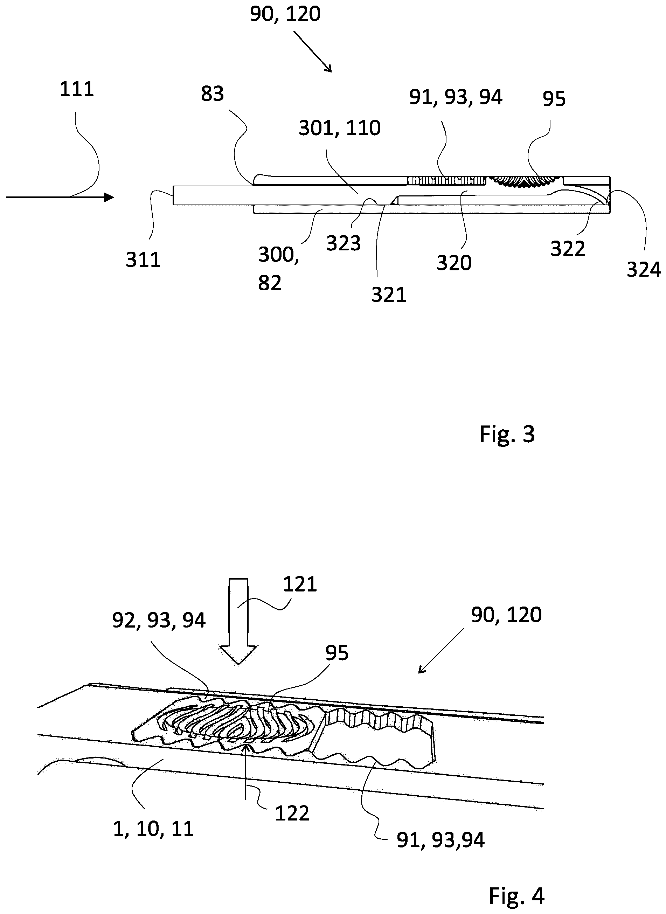

As fig.1 and fig.2 show, the number of teeth 94 of the first part 300 of the ratchet system 90 exceeds the number of teeth 94 of the second part 301 of the ratchet system 90. As a consequence, various locking positions are enabled along a slide-in direction 111, wherein the locking position that is most to the right is selected here, such that the longitudinal extension of the ratchet system 90 is minimized. The slide-in direction 111 is illustrated in fig.3.

Fig. 3 depicts a cross sectional view of the ratchet system 90 in the same embodiment as in fig 1. It reveals that the first serration 91 of the first part 300 of the ratchet system 90 is provided by a retainer 82, and the second serration 92 is provided by an insertion element 110, whereby the insertion element 110 can be inserted into the retainer 82 through an insertion opening 83 along the slide-in direction 111.

In the illustrated state, the insertion element 110 is completely inserted into the retainer 82 and the serrations elements 93 in the shape of teeth 94 are in a state of positive locking 120 in a plane of engagement. The button 95 is located on top of a bridging section 320 of the insertion element 110 and conceals the view on the second serration 92.

The retainer 82 encloses the insertion element 110, such that at least one rotational degree of freedom of the inserted insertion element 110 around an axis perpendicular to the slide-in direction 111 and parallel to the plane of the engagement of the serration elements 93 is blocked.

Moreover, the insertion element 110 has a portal-shaped form and has a first support element 321 located ahead the bridging section 320 and a second support element 322 located behind the bridging section 320 in relation to the slide-in direction 111. The support elements 321,322 support the insertion element 110 on two support parts 323,324, wherein the support parts 323,324 are integral parts of one single surface of the retainer 82. Both support elements 321,322 are protruding from the bridging section 320.

When a person's finger presses the button 95, the bridging section 320 and the second support element 322, located at the end section of the insertion element 110, undergo elastic deformation. As a result, the first and second serration 91,92 disengage and the insertion element 110 can be moved along a slide-in direction 111 as long as the button 95 remains pressed.

Fig. 4 to 7 show different states of the ratchet system 90, illustrating a method of changing locking positions. Thereby, the first serration 91 is part of a cuff 1 and the second serration 92 is part of a device according to the invention.

Once the insertion element 110 of the device 100 is inserted in the insertion opening 83, the user has to press the button 95, shown in fig. 4 to 7, and slide the insertion element 110 of the device 100 into the lower shell 11, while keeping the button 95 pressed. Here, the button 95 is a special design on the insertion element 110 of the device 100, which slides into the lower shell 11. The user can then release the button 95 when the desired position is reached.

For fixation of different positions of the device 100 with regard to the cuff 1, the orthotic system comprising the cuff 1 and device 100 comprises the ratchet system 90. The ratchet system 90 comprises the first serration 91 provided by the lower shell 11 of the cuff 1, as well as the second serration 92 provided by the insertion element 110 of the device 100 and/ or at the button 95. Both components 91, 92 of the ratchet system 90 feature serration elements 93, having a shape like teeth 94. In one embodiment, these teeth 94 may have a rounded shape.

The serration elements 93 of the first serration 91 and the second serration 92 are complementary to each other in form and size.

Furthermore, the device 100 can slide along the slide-in direction 111 parallel to an arm axis. Depending on the activity and intensity, different positions of the device 100 along the y-axis might be required. This allows for the same design to be used by users with different arm lengths.

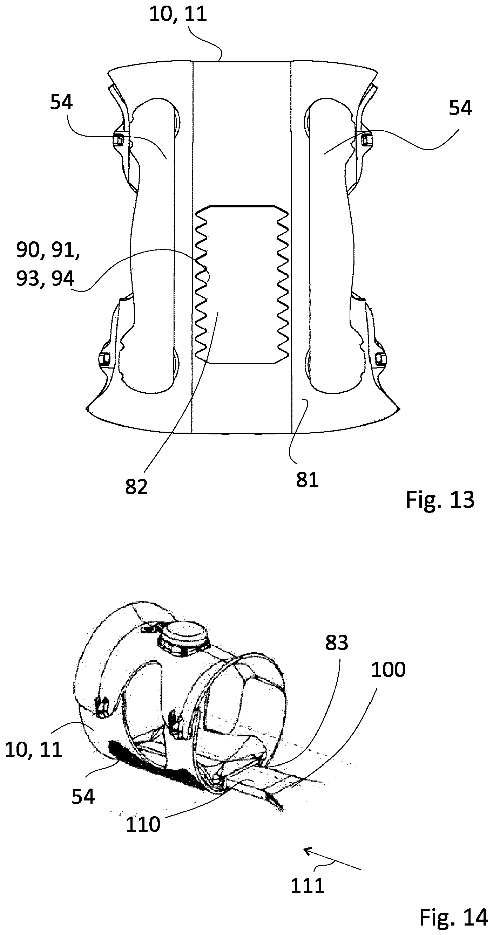

The distance between the area of interest of the device 100 and the insertion opening 83 of the lower shell 11 as seen in fig. 14 can be adjusted by means of the ratchet system 90.

The number of positions of the device 100 is determined by the number of serration elements 93 on the first serration 91 and the second serration 92 of the ratchet system 90.

Fig. 4 to 7 show a method for changing the position of the device 100 with respect to the cuff.

In fig. 4 to 7 a part of the lower shell 11 or the cuff base 10 of the cuff 1 is shown, as well as a part of the insertion element 110 of a device 100, which is not shown completely.

At the insertion element 110 the button 95 is provided.

In the retainer 82 of the lower shell 11 the first serration 91 of the ratchet system 90 is provided, comprising a plurality of serration elements 93 in the shape of teeth 94, respectively.

At the insertion element 110 and/ or at the button 95 the second serration 92 is provided, comprising also a plurality of serration elements 93 in the shape of a teeth 94, respectively.

The serration elements 93 of the first serration 91 and of the second serration 92 are arranged in rows, respectively, and are complementary to each other in form and size.

Fig.4 shows a state in which the serration elements 93 of the first serration 91 and the second serration 92 engage with each other. Thus, a positive locking 120 is established between the lower shell 11 and the insertion element 110. This engaged state is maintained by means of a force 122 acting on the insertion element 110 due to elasticity of the insertion element 110.

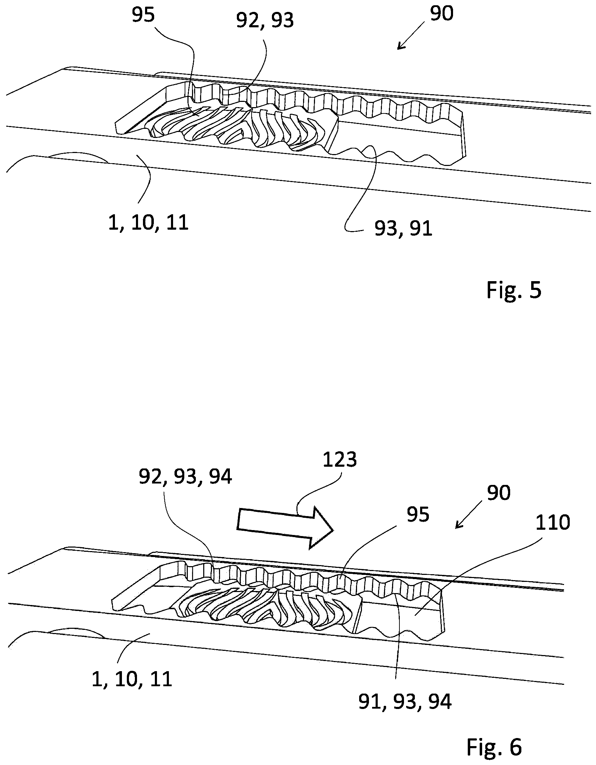

When the button 95 is pressed by a pressing force 121, for instance manually, the button 95 and therefore the insertion element 110 is pressed inward against the elastic force 122, wherein the serration elements 93 of the first serration 91 and of the second serration 92 are moved out of their engagement, so that the positive locking 120 does not exist anymore, as shown in fig. 5.

When the insertion element 110 has been moved inward, by applying a moving force 123 on the button 95, the insertion element 110 can be moved parallel to the extension of the rows of serration elements 93 of the first serration 91 and of the second serration 92. Thus, the position of the insertion element 110 and therefore the position of the device 100 with respect to the cuff 1 can be adjusted, see fig. 6.

When the foreseen position has been reached, the button 95 can be released and due to elastic force 122 the insertion element 110 as well as the button 95 moves so that the serration elements 93 of the first serration 91 and of the second serration 92 engage again, shown in fig. 7.

Fig. 8 shows a device 100 in a perspective view in accordance to the invention, comprising the second part 301 of the ratchet system 90. The device 100 comprises a limb connection system 210 which has an elongated shape. Part of the limb connection system 210 is an insertion element 110 that is equipped with the second serration 92 of a ratchet system 90 comprising serration elements 93 in the shape of teeth 94 and a button 95.

There are two rows of teeth 94 that are located on top of the insertion element 110. The row of teeth 94 are facing into opposite directions, pointing sideways away from the insertion element 110 and away from the button 95.

The second serration 92 of the ratchet system 90 can be disengaged from a positive locking 120 in a convenient way by pressing the button 95.

The device 100 further shows a mechanical holding mount 220, which has a cavity 221 to establish a form fitting connect with an object. A belt 230 with an integrated hook-and-loop fastener 231 is connected at two sides of the cavity 22. The form of the belt 230 illustrates how an object can be wrapped in order to fix its position. Thereby the belt 230 eventually applies tensional force to establish a force-fitting connection to the object.

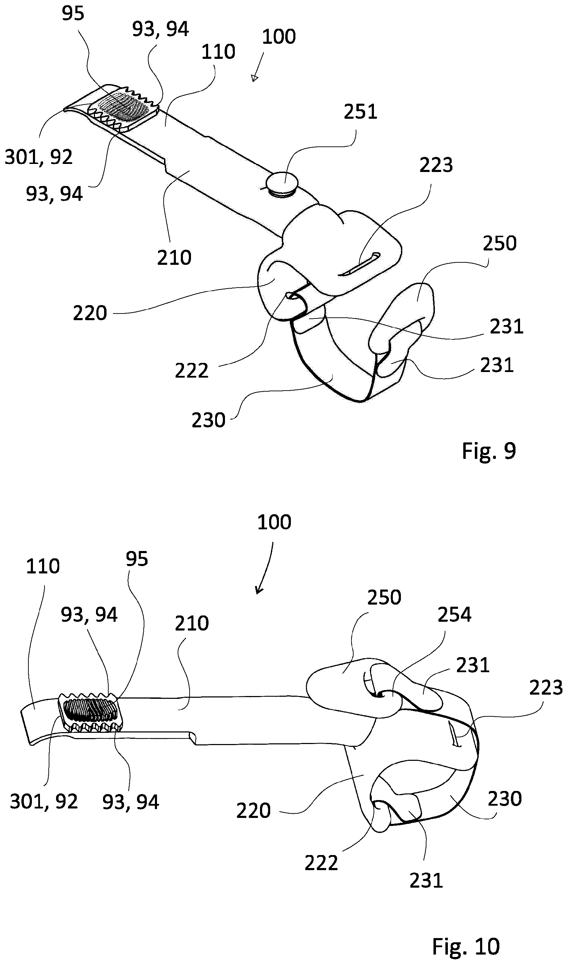

As shown in fig 9, another embodiment of the device 100 comprises an identical limb connection system 210, but a different mechanical holding mount 220. Moreover, a different connection system of the belt 230 is demonstrated. Both end sections of the belt 230 comprise a hook-and-loop fastener 231, respectively. The belt 230 is shown is opened state.

One end section of the belt 230 is threaded through a first slit 222 at the mechanical holding mount 220 and attached to itself, while the other end section is attached in a similar way to a first mounting element 250. The second mounting element 251 is located at the limb connection system 210. In order to close the belt 230, the first and the second mounting elements 250, 251 can be brought into a form-fitting connection.

Additionally, the mechanical holding mount comprises a second slit 223, where the belt 230 optionally can be connected or which can be used as guidance for the belt 230.

Fig. 10 shows the device 100 in the same embodiment as shown in fig. 2, wherein the belt 230 is in a closed state. The first and the second mounting elements 250, 251 are depicted in a connected state. Thereby, the first mounting element 250 blocks the view onto the second mounting element 251. The first mounting element 250 further comprises a bar 254, where the belt 230 is looped around and connected to itself by means of the hook-and-loop fastener 231. The opposite end section of the belt 230 is also equipped with a hook-and-loop fastener 231, which is connected to the first slit 222 of the mechanical holding mount 220 in a similar manner. The slit 223 either can be used for guidance purposes of the belt 230 or can be used to fixture one end section of the belt 230.

The first part 300 of the ratchet system 90 can be part of a cuff 1 that is attachable to a limb in accordance to the invention. Fig. 11 to 14 show such a cuff 1.

Insertion of a limb, for instance a forearm, into the cuff 1 can be difficult as, in most cases, there is an enlargement at the beginning of the palm. This makes it difficult to insert the arm into the cuff 1 from the proximal side. In order to facilitate the insertion of the arm into the cuff 1 on the proximal side, the design is divided into a cuff base 10 in form of a lower shell 11 and a fixation element 20, which can be a cuff top 30 in form of an upper shell 31.

As shown in the embodiment of fig. 11, the shells 11, 31 can be separated as two distinct elements. The lower shell 11 can in this case be set radially to the axis of the arm. The upper shell 31 can then be coupled to the lower shell 11 to create a closed system around the forearm.

The number of shells 11, 31 can be larger than two and rise to three, four or more, but that can be the case only if they can be linked in such a way that the user does not need to hold them all in position before tightening them together around their forearm.

The way in which the shells can be connected to each other should be easy and effortless. In the embodiment shown in fig. 11, the upper shell 31 as the fixation element 20 is connected to the lower shell 11 by means of the lacing system 60.

The lacing element 61 in form of a cable, which is connected with a lacing knob 62. By turning of the lacing knob 62, tension is generated in the lacing element, which causes the tightening of the lacing system 60.

As the tension generated in the lacing system 60 increases, the circumference becomes reduced, allowing the lacing system 60 to be tightened around the forearm which results in better transmission of the forces exerted on the cuff 1.

The lacing system 60 may comprise a common part 54 which provides a plurality of form fit means. These can be used to interlock with additional form fit means provided at the lower shell 11 establishing a form fit connection. In fig 11, 13 and 14 the form fit means are not illustrated, but the common part 54 is shown, respectively.

In fig. 12, a different embodiment of the cuff 1 is shown. In this embodiment, the cuff 1 provides a cuff base 10 in form of a lower shell 11 and a fixation means 20 in form of a strap system 70. The strap system 70 comprises two straps 71 which are led through cutouts 73 of the cuff base 10. The straps 71 are provided with a hook-and-loop system 72, respectively.

By tensioning the straps 71 and fixation of the hook-and-loop system 72, the strap system 70 can be tightened around the forearm.

The embodiment of the cuff 1 comprising a strap system 70 is not restricted to the embodiment shown in fig. 12.

As in the embodiments shown in fig. 11, cushioning material 80 is applied in the cuff base 10.

Another aspect of the design is its modularity.

The system allows for the connection of different modules to best meet the needs of the users. Especially, it can be connected to the device 100.The device 100 can be inserted into the lower shell 11 of the cuff 1.

Fig. 14 depicts the insertion of the device 100 into the cuff 1, wherein the device 100 is only partly illustrated. As shown, the insertion element 110 of the device 100 can be directly inserted along a slide-in direction 111 into a retainer 82, which is provided in the cuff base 10 through an insertion opening 83 which in turn is arranged at the distal end of the cuff 1, shown in fig. 13. Positive locking 120 of the ratchet system 90 allows the transfer of loads along the slide-in direction 111

The device 100 can be removed from the cuff 1, when necessary, for example, if the targeted activity changes, requiring another module.

The orthotic system allows for outdoor activities and can be used for muscle-strengthening sessions or fitness training It can also be used in the water, for example, when kayaking. These activities require a critical degree of freedom so as not to restrict the movement of the upper limbs.

### List of reference signs:

- 1: cuff
- 10: cuff base
- 11: lower shell
- 20: fixation element
- 30: cuff top
- 31: upper shell
- 54: common part
- 60: lacing system
- 61: lacing element
- 62: lacing knob
- 70: strap system
- 71: strap
- 72: hook-and-loop system
- 73: cutout
- 80: cushioning material
- 81: distal end
- 82: retainer
- 83: insertion opening
- 90: ratchet system
- 91: first serration
- 92: second serration
- 93: serration element
- 94: teeth
- 95: button
- 96: button cavity
- 97: structured surface
- 100: device
- 110: insertion element
- 111: slide-in direction
- 120: positive locking
- 121: pressing force
- 122: force
- 123: moving force
- 210: limb connection system
- 220: mechanical holding mount
- 221: cavity
- 222: first slit
- 223: second slit
- 230: belt
- 231: hook-and-loop fastener
- 250: first mounting element
- 251: second mounting element
- 254: bar
- 300: first part of the ratchet system
- 301: second part of the ratchet system
- 310: first connection element
- 311: second connection element
- 320: bridging section
- 321: first support element
- 322: second support element
- 323: first support part
- 324: second support part

## Claims

1. Ratchet system (90), comprising a first part (300) of the ratchet system (90) equipped with a first serration (91) and a second part (301) of the ratchet system (90) equipped with a second serration (92), wherein the two serrations (91,92) comprise serration elements (93) that are at least partially complementary in terms of shape, number, and/or size, and wherein the first part (300) of the ratchet system (90) has a retainer (82) with an insertion opening (83) and is equipped with the first serration (91), and the second part (301) of the ratchet system (90) has an insertion element (110) equipped with the second serration (92), such that after insertion of the insertion element (110) through the insertion opening (83) along a slide-in direction (111) into the retainer (82), the two serrations (91,92) can interlock and can block at least one translational degree of freedom of the insertion element (110) along the slide-in direction (110) with respect to the retainer (82).

2. Ratchet System (90) according to claim 1, wherein the first serration (91) at the first part (300) of the ratchet system (90) and second serration (92) at the second part (301) of the ratchet system (90) comprise a set of teeth (94), respectively.

3. Ratchet system (90) according to at least one of the preceding claims, **characterized in that** the insertion element (110) has a transversal extension and a longitudinal extension longer than the transversal extension and can be inserted along its longitudinal extension into the retainer (82), wherein the retainer (82) comprises at least two opposing surfaces, which enclose or can enclose the insertion element (110), such that at least one translational degree of freedom of the insertion element (110) perpendicular to the slide-in direction (111) with respect to the retainer (82) is at least partially blocked or can be at least partially blocked by the retainer (82).

4. Ratchet system (90) according to at least one of the preceding claims, wherein the first serration (91) and the second serration (92) have serration elements (94) that are arranged along the slide-in direction (111) in such a way that more than one locking position and/or insertion length of the insertion element (110) in the retainer (82) can be realized along the slide-in direction (111).

5. Ratchet system (90) according to at least one of the preceding claims, **characterized in that** the insertion element (110) can at least partially be displaced and/or deformed due to the application of a pressing force (121) induced manually to the insertion element (110), so that an engagement of the first serration (91) and the second serration (93) can be prevented or dissolved.

6. Ratchet system (90) according to claim 5, wherein the insertion element (110) is adapted in such a way that the manually applied pressing force (121) measures at least 3 N, which elastically bends and thus displaces at least a section of the insertion element (110) and thereby prevents or dissolves the engagement between the first serration (91) and the second serration (92), wherein the Young's modulus of the insertion element (110) is between 0.5 GPa and 7 GPa.

7. Ratchet system (90) according to at least one of the claims 5 and 6, wherein the insertion element (110) comprises an optically and/or haptically recognizable button (95), designated for applying the pressing force (121) in the insertion element (110) in order to displace and/or deform the insertion element (110) at least partially, thereby preventing or dissolving the engagement between the first serration (91) and the second serration (92).

8. Ratchet system (90), according to claim 7, **characterized in that** the second serration (92) provided by the insertion element (110) comprises two rows of serration elements (93), that are aligned parallel to one another and parallel to the slide-in direction (111), wherein the button (95) is located in between these two rows and that the first serration (91) provided by the retainer (92) also comprises to rows of serration elements (93) that are parallel to one another and parallel to the slide-in direction (111).

9. Ratchet system (90) according to at least one of the preceding claims, **characterized in that** the insertion element (110) is at least partially provided by a material having a Young's modulus between 0.5 GPa and 7 GPa so that after elastic deformation of the insertion element (110) due to elastic behaviour of the insertion element (110) at least one section of the insertion element (110) is movable such that the second serration (92) of the insertion element (110) is brought into positive locking (120) with the first serration (91) of the retainer (82).

10. Ratchet system (90) according to at least one of the preceding claims, **characterized in that** the insertion element (110) has a bridging section (320), comprising the second serration (92), a first support element (321) and a second support element (322) located ahead and behind the bridging section (320) in relation to the slide-in direction (111) for supporting the insertion element (110) on support parts (323,324) provided by the retainer (82), with the support elements (321,322) protruding at an angle from the bridging section (320) with respect to the slide-in direction (111), respectively, wherein the first and second serrations (91,92) are configured such that their serration elements (94) are interlocked in an engaged state in a plane of engagement, wherein the retainer (82) forms an enclosure for the insertion element (110), blocking at least one rotational degree of freedom of the inserted insertion element (110) around an axis perpendicular to the slide-in direction (111), wherein the bridging section (230) and/or the second support element (322) located at the end of the bridging section (230) are provided by a material having a Young's modulus between 0.5 GPa and 7 GPa, so that when a manual force is applied to the bridging section (230), the bridging section (230) and/or said second support element (322) is able to elastically deform, resulting in the disengagement of the first and second serrations (91,92).

11. Ratchet system (90) according to at least one of the preceding claims, wherein the ratchet system (90) is an interface which comprises a first connection element (310), designed to mechanically connect the first part (300) of the ratchet system (90) to an orthotic accessory connectable to a person's limb or designed to mechanically connect the first part (300) of the ratchet system (90) to a module for establishing a connection to an object, and further comprises a second connection element (311), designed to mechanically connect the second part (301) of the ratchet system (90) to the respective other part of orthotic accessory or module, in order to enable, that the orthotic accessory and the module can be repeatable connected and disconnected.

12. Orthotic system, comprising a ratchet system (90) according to at least one of the claims 1 to 11 and an orthotic accessory connectable to a limb.

13. Orthotic system according to claim 12, wherein the orthotic accessory is part of a cuff (1), connectable to a limb.

14. Orthotic system, according to claim 13, wherein the cuff (1) is provided by a cuff base (10) in form of a lower shell (11) and a fixation element (20) which can be combined or is combined with the lower shell (11) into a sleeve attachable to a limb through direct or indirect interlocking with each other, wherein the lower shell (11) and the fixation element (20) provide a system that can be tightened around the limb by reducing a circumference formed by the lower shell (11) and the fixation element (20), such that load transfer from an accessory to the limb is enabled.

15. Orthotic system according to one of the claims 12 to 14, wherein the second part (301) of the ratchet system (90) is provided by a device (100) for establishing a form-fitting and/or force-fitting connection to an object that comprises a mechanical holding mount (220), where the object can be positioned, such that load transfer from the object to the limb is enabled, and at least one belt (230) mechanically connected to said holding mount (220), wherein the belt (230) is configured to be partially wrapped around the object positioned in the mechanical holding mount (220) in order to fix the object in at least one translational degree of freedom.
